# EUROPEAN PATENT SPECIFICATION

(11) **EP 3 787 578 B1**
(45) Date of publication and mention of the grant of the patent: **07.09.2022**
(21) Application number: 19729614.8
(22) Date of filing: 03.05.2019
(51) Int. Cl.: A61F 9/007

(54) **AN EYE TREATMENT DEVICE**
AUGENBEHANDLUNGSVORRICHTUNG
DISPOSITIF DE TRAITEMENT DES YEUX

(30) Priority: 04.05.2018 DK PA201870271
(43) Date of publication of application: 10.03.2021
(73) Proprietor: JULIAN, Kim, 2920 Charlottenlund (DK)
(72) Inventor: JULIAN, Kim, 2920 Charlottenlund (DK)
(74) Representative: Holme Patent A/S
(86) International application number: PCT/DK2019/050137
(87) International publication number: WO 2019/210927

(56) References cited:
- WO-A1-2014/018651
- WO-A1-2018/039729
- US-A1- 2012 065 556
- US-A1- 2014 214 062
- US-A1- 2015 182 415
- US-A1- 2015 216 722

## Description

The present invention relates to an eye treatment device arranged for treatment of an eye disease.

Humans may be exposed to various eye diseases, and sometimes to diseases in the eyelid region. Such diseases can, for example, be inflammation of the eyelid, eyelid infections, meibomian gland dysfunction, blepharitis, and dry eye syndrome, which among others may cause red eyes, that the skin of eyelids shells, and that debris, crusts and/or cysts are formed at the base of the eyelids. This will in turn make the eyes feel sore, burning and/or irritated, impairing the patient's vision. Meibomian gland dysfunction is traditionally treated with wet compresses and lid hygiene for treating the obstructed meibummian gland, as well as with antibiotics and antiinflammatory agents. However, such treatments are complicated and unpleasant for the patient, making it difficult to obtain the complete relief of symptoms.

In a similar manner the conventional treatment for blepharitis will often consist of a medical treatment combined with hygienic treatment for regular removal of debris that have accumulated along the base of the eyelashes. It is important that the accumulated debris or be removed at regular intervals, otherwise such eye disease may worsen. Such hygienic treatment often takes place by the person first softens the debris, and then in a second step mechanically removes the debris, by scrubbing the eyelashes. The softening process step may be done by applying a cloth or pad with an appropriate liquid absorbed in it to the eyelash region. Thereafter the soften debris can be removed by scrubbing with a cotton swab, a fingertip or a pad over the eye. Removal of this debris is critical to both healing the eye and preventing a resurgence of the disorder. Without proper, regular removal of accumulated debris, such ocular disorders regularly worsen despite periodic treatments. However, this approach is for various reasons not optimal and therefore new ways to carry out the hygiene treatment have been proposed. Thus, in WO 2014/018651, a method and a device for treating an eye disease are suggested. Said device comprises a rotating swab connected to an electromechanical device that mechanically can remove debris from the base of the eye lashes. The disadvantage of this known device is that it only gives a solution to the second treatment step of mechanically removing the debris from the eyelid, and in order to achieve this the device have to have a very high rotational speed (e.g. above 300 rpm), and the known device therefore have to be operated at trained professionals only. Furthermore, such device cannot be used for massaging the meibomian glands.

A device according to the preamble of claim 1 is known from the document US 2012/065556.

It is therefore a first aspect of the present invention to provided a device for treatment of an eye disease, in which the two process steps of softening and scrubbing the debris, crusts and/or cysts may be done in a single process step.

In a further aspect is provided an eye treatment device according to the invention that can be used for massaging the meibomian glands.

In a further aspect is provided an eye treatment device according to the invention, by which the time consumption for treatment of the eye is reduced.

In a further aspect is provided an eye treatment device according to the invention, which is inexpensive to manufacture.

In a further aspect is provide an eye treatment device according to the invention, which is simple and easy to use, and which is suitable for home-treatment.

The novel and unique whereby these and other aspects are achieved according to the present invention by providing an eye treatment device arranged for removing debris from an eyelid margin, said device comprises a swab operatively connected to a housing that accommodates a drive unit, said drive unit being arranged for moving the swab relative to said housing, and wherein said device comprises heating means arranged for heating the swab.

The eye treatment device ensures that when a person places the heated swab at the eyelid region in contact with the debris whereby the debris will be heated and accordingly softened. As the swab is arranged to move relatively to the housing e.g. in an rotating, vibrating and/or reciprocating movement, the debris will be mechanically removed in the same process step as the softening of the debris. This means that the user in a single operation e.g. by moving the eye treatment device along or at the margin/base of the eyelid, effectively can cleanse the margin of the eyelid and the base of the eyelashes and/or massage the meibomian glands thereby mechanically open and dilate the natural orifices and ducts of the meibomian glands to remove abnormal meibum secretions. Thus, the device according to the invention may remove debris and/or massage the meibomian glands and/or provide another kind of treatment much easier, faster and in a more secure manner than using e.g. the known devices and methods. Furthermore, the time consumption for treatment of the eye is reduced, thereby providing an eye treatment device which can be used for regular, effective and hygienic home treatment.

The inventor of the present inventing has found that when eye treatment device is arranged for heating the swap to a predefined temperature between 33°C and 45°C, and especially advantageously embodiment is provided, in which the debris is softened to a desired degree during use but without risking that the user becomes burned. The optimal temperature depends on the desired use of the eye treatment device, but is preferably between 40°C - 43°C. However, if the eye treatment device e.g. is used for treating meibomian gland disease, the predefined temperature may alternatively be about 34°C, as said temperature has proven highly efficient in the treatment of said disease.

The eye treatment device preferably comprises temperature controlling means arranged to heat the temperature of the swap to the predefined temperature and/or maintain the swap at said temperature. Said heat controlling means may e.g. be a thermostat which will turn the heat on/off depending on the temperature of the swap; one of more temperature sensors; control units etc. This will also ensure that the temperature will not increase beyond an upper temperature limit, i.e. said controlling means will function as a safety switch. Such temperatures controlling means are will known in the art, and any relevant means may be included in the eye treatment device according to the invention as long as it is capable of providing and maintaining the predetermined temperature of the swap.

In a preferred embodiment the temperatures controlling means further comprises a temperature adjustment means arranged for individually adjusting the temperature of the swap, e.g. such that the user can increase or reduce said temperature if desired. Such temperature adjustment means may be any conventional means which ensures that the user e.g. can choose between a number of preset temperatures, such as 34°C, 37°C, 39°C and 41°C, and/or that the user can select any desired temperature between an upper and a lower preset temperature. Irrespectively of how the heat controlling means and/or temperature adjustment means is arranged, it is preferred that the temperature of the swab cannot exceed a temperature above 45 °C and/or be lowered below 33 °C, thereby ensuring e.g. that the debris is softened to a desired degree without risking that the user is burned.

The eye treatment device may in a preferred embodiment comprise indication means, which e.g. visually and/or aurally indicates if and when the predetermined temperature has been reached, and/or if further heating is required during operation. It is however preferred that the heat controlling means is arranged for automatically ensuring that the temperature is maintained at the selected temperature during use.

In one embodiment the eye treatment device further comprises an ultrasonic means, i.e. a means arranged for generating vibrations at ultrasonic frequencies e.g. from 10 - 40 kHz. The generated vibrations will then aid in removing the debris from the margin of the eye lid and/or massage the meibomian glands when the swab is moved along the margin/base of the eyelid. In a preferred embodiment the ultrasound is arranged for agitating a fluid which e.g. has been applied to the margin of the eye lid, thereby increasing the efficiency of the ultrasonic means.

The swap may in one embodiment be directly coupled to the housing, however in order to ensure that the eye treatment device can be operated without e.g. the housing abutting the chin, the swap may be coupled to the housing by means of an extension member. Said extension member may in a preferred embodiment be releasably coupled to the housing, such that the user can select an extension member having a desired length.

In one embodiment the swab constitutes a part that is inseparable from the extension member, which means that the both parts must be replaced when the swap is worn or too dirty to be used. This is a relatively expensive solution. Therefore, the extension member is preferably arranged such that the swap can be releasably and securely attached to the extension member. This provides a simple and very cheap solution because only a very small and cheap part needs to be replaced, when the swab is e.g. used once.

If the extension member is a substantially rigid rod, a swap e.g. made of sponge material can easily be held in place by frictional forces. However, the swap may e.g. comprise a first connector arranged for being releasable coupled to a second connector. Preferably both the first and second connector have a mainly cylindrical shape adapted to provide a tight fit when the second connector extend at least partly into the first connector. Such a configuration will ensure that the swap easily can be replaced after use, and that a new swab easily can be applied to the rigid rod without having to concentrate about a specific orientation of the swab. However, other configurations are also contemplated within the scope of the present invention, e.g. an embodiment in which a swap of a spongy body simply is pressed onto the extension member.

In order to heat the swab, the eye treatment device according to the invention comprises at least one heating means arranged for heating the swab to the predefined temperature. The heating means may be constituted by any suitable means that can ensure that the swab is heated. In one embodiment, the heating means may be arranged immediately at the swab and be provided with electrical power through power lines drawn in the rigid rod, which in such an embodiment should be provided with a channel for accommodating the power lines. However, in an embodiment of the present invention the heating means may be arranged inside the housing and the rigid rod is made of heat conductive material. In this way, the heating means is very well protected from being destroyed and the heat from the heating means is led to the swab via the rigid rod.

The heating means may be constituted of any suitable means, however in an embodiment of the present invention the heating means may comprise electrical heating means and an electrical source for supplying electricity to the electrical heating means. This provides a safe and reliable device for heating the swab, which ensures a uniform heating of the swab.

The rigid rod may be heated in different ways, either directly or indirectly, mechanically by friction or electrically. Thus, in one embodiment of the eye treatment device according to the invention the rigid rod may be an electrical resistance heating element, which is connected directly to the electrical source, preferably by means of sliding contacts that allows the rigid rod to be rotated, vibrated and/or reciprocated. In this way, a very good and uniform heating of the rigid rod can be achieved every time the device is used.

In another embodiment the heating means may comprise a heating element which surrounds part of the rigid rod. This provides a cheaper solution where the swab and also the rigid rod may be replaced relatively cheaply. This will be an advantage in cases where the also the extension member can be replaced e.g. due to malfunction, damage or because it becomes worn to be used again.

The heating means may comprise a switch to turn on and off the heating means. The switch may be controlled by a motion sensor, however, in an embodiment of the present invention the switch may be controlled manually by operating an on/off-button placed on the housing. In this way, it is ensured that the heating means is not turned on or off inadvertently.

The drive unit for moving the swab relative to the drive unit, i.e. rotating the swab, may comprise any suitable means arranged for moving the swab. In a preferred embodiment the apparatus is arranged for moving, e.g. rotating the extension member, and thereby the swab. However, the swab may also be rotated independently of the extension member. Accordingly, the drive unit may comprise a mechanical arrangement, such as a spring device which is tighten each time the eye treatment device is to be used. However in an embodiment of the present invention the drive unit for moving the extension member and/or swab relative to the drive unit may comprise an electrical motor and an electrical source for supplying electricity to the electrical motor. This provides a reliable solution that is immediately ready for use.

The extension member may be connected to the electrical motor of the drive unit via any appropriate means, such as a gearing. However, in an embodiment of the present invention the extension member may be connected directly to the electrical motor of the drive unit via a shaft. In this way, a good and reliable drive system for moving the swab relative to the drive unit, i.e. rotating, vibrating and/or reciprocating the swab is obtained.

Because the person using the eye treatment device to remove debris and various crusts from the eyelash or the eyelid typically will move the swab back and forth alone the margin of the eyelid and the base of the eyelashes, and it is preferred that the swab is able to be rotated in both directions. Accordingly, in an embodiment of the present invention the electrical motor of the drive unit may be rotatable in both directions. Hereby, the swab, regardless of the direction it is moved, may always be rotated in a direction that ensures that debris, crusts and/or cysts and the like are pushed forward in front of the swab.

It is furthermore preferred that the swab has a size/dimension which ensures that it easily can be moved along the margin/base of the eyelid without causing damage to the eyeball. It is furthermore preferred that said size/dimension is selected such that if the user is using the eye treatment device for self treatment, said swab will not block the users vision during use. It is accordingly preferred that the swab is relatively small with an substantially tubular shape preferably with a rounded end. Said tubular swab may have a diameter in the area between 0.2 cm and 1.0 cm, and preferably around 0.5 cm. The length of the swab may e.g. be between 0.5 and 2.0 cm.

The electrical source for supplying electricity to the drive unit and/or to the electrical heating means may be the power grid or a battery or both. In an embodiment of the present invention the electrical source is a battery, because the eye treatment device can thus be used independently of the access to the power grid. The battery is preferably a rechargeable battery, which may be recharged by means of the power grid, e.g. by means of power outlet, a UBS-port or the like. In this way, the battery may be charged when there is access to the power grid and otherwise the device may be used independently.

The drive unit comprises a switch to turn on and off the drive unit. The switch may like the switch for turning on and off the heating means be controlled by a motion sensor, however, in an embodiment of the present invention the switch may be controlled manually by operating a button.

It is the treatment member of the swab that is in contact with the eyelash or eyelid during the treatment of the eye, however if the swab unintentionally comes into contact with the eye ball, the swab may in a preferred embodiment be made of a cotton wadding or sponge material ensuring that this will not feel uncomfortable for a person using the eye treatment device. By sponge material is meant a material that is soft, porous and resilient. Hereby is obtained that the swab can be used without major discomfort for the person utilizing the eye treatment device and that tear liquid as well as debris, crusts and/or cysts are absorbed by the treatment member.

Additionally, a swab made e.g. of a sponge material will also be able to absorb a treatment liquid that can be used to either soften the debris and/or for providing a medical treatment. In this way, an eye disease can be treated more efficiently. Any liquid solution capable of disinfecting and/or loosening the debris to help removal of the debris may be used. A suitable treatment liquid may e.g. be physiological saline and/or a pharmaceutical composition aiming at treating the specific eye condition.

It is preferred that the swab has a substantial ball or cone shape, as this ensures that the eye treatment device can be controlled very accurately along the margin of the eyelid and/or the base of the eyelashes, e.g. effectively massaging the meibomian glands, i.e. the change of unintentionally touching the eye ball is reduced.

In order to ensure that the eye ball is not damaged if the swab unintentionally touches said eye ball during use of the eye treatment device according to the invention, it is preferred that the swab is rotated at a relatively low speed, e.g. between 50 and 150 rpm, preferably between 100 and 110 rpm. This will also ensure that the eye treatment device is suitable for home treatment, as the low rotational speed will reduce the users anxiety to place an object close to the eye, and will accordingly increase the users ability to specifically target debris deposits. In a preferred embodiment the eye treatment device comprises rotation control means arranged for allowing adjustment of the rotational speed during use, e.g. such that the user can increase or reduce said speed if desired. Such rotation control means may be any suitable means arranged such that the user can select between a number of preset rotational speed values, e.g. 50 rpm, 100 rpm and 150 rpm, or individually select any rotational speed between an upper and a lower preselected speed limit. Irrespectively of how the rotation control means is arranged, it is preferred that the swab is rotated at the relatively low speed as defined above, such that if the swab unintentionally touches the eye ball during use, said swab will not damage the eye ball.

Also disclosed but not being part of the present invention, is a method of using the eye treatment device, this method comprises,
- providing an eye treatment device according to the invention,
- heating the swab to a temperature between 33 °C and 45 °C, e.g. about 34°C or between 40°C - 43°C,
- rotating the swap, and
- move said along the margin of the eyelid and/or the base of the eyelashes.

In a preferred embodiment of said method, the swab is placed in contact with a treatment liquid such that said swab absorbs at least some of said liquid.

The eye treatment device according to the invention not only has a simple and inexpensive construction, but also has a simple and user-friendly design, making it extremely easy to operate with a single hand ensuring that the eye treatment device can be unassisted used at home.

The invention will be explained in greater detail below, describing only exemplary embodiments of the eye treatment device with reference to the drawing, in which
Fig. 1 is a perspective top view of an eye treatment device according to the invention,
fig. 2 is a side view of the eye treatment device in fig. 1,
fig. 3 is a schematic side view of the eye treatment device with one side of the housing removed, and
fig. 4 is a drawing of the eye treatment device of fig. 1 treating a lower eyelid of an eye.

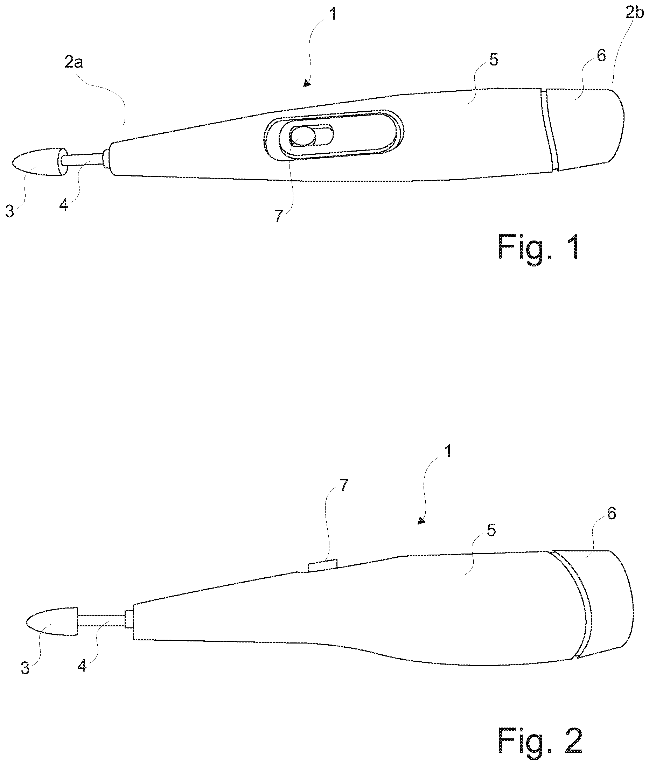

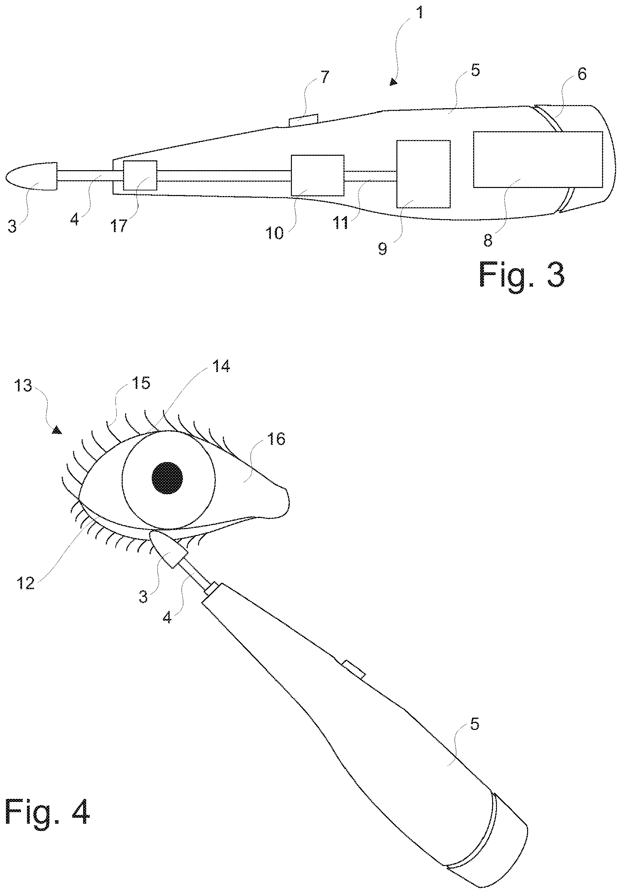

In figs. 1 and 2 is shown an eye treatment device 1 for cleaning of eyelashes and eyelid margins in the treatment of an eye disease. The eye treatment device 1 comprises at its proximal end 2a, a swab 3 and a substantially rigid rod 4. Further, the eye treatment device 1 comprises a housing 5, which is formed to act as a handle. The housing 5 accommodates essential parts of the eye treatment device, as it will be described in further details with reference to fig. 3. Further, the housing 5 comprises a lid 6 at a distal end 2b. The lid 6 opens into a battery section 8 in order to exchange the batteries. Further, the eye treatment device comprises a manually operated switch button 7 with which the eye treatment device may be turned on and off.

The eye treatment device 1 has a size which means that it fits well in the hand of the person performing the treatment of the eye.

Fig. 3 schematically shows the inside of the housing 5. Thus, the eye treatment device 1 is shown to comprise the battery section 8, to which access is achieved via the lid 6. The batteries used are preferable rechargeable batteries. The eye treatment device 1 accommodates two rechargeable batteries of type AAA, However, the eye treatment device 1 may also be arranged for being connected to a charging station.

Further, the eye treatment device 1 comprises a drive unit 9 in form of an electrical motor 9 for rotating the swab 3, and heating means 10 for heating the swab 3. The rigid rod 4 of the swab 2 is connected to the electrical motor 9 via a shaft 11 and is thereby rotatable by the electrical motor 9. Furthermore, the rigid rod 4 of the swab 2 is mounted and journalled in a bearing 17 arranged close to the end of the housing 5 comprising the swap 2. The eye treatment device 1 may also include means for vibrating and/or reciprocating the swab 2, however such means is not shown in fig. 3.

The rigid rod 4 is made of any heat conductive material, such as any metal as iron or steel. The heating means 10 comprises an electrical heating element and a temperature controlling means arranged to heat the temperature of the rigid rod 4 and the swap 3 to the predefined temperature of e.g. between 40 to 43 °C and maintain the swap at said temperature.

In an alternative not shown embodiment, the rigid rod 4 of the swab 2 may be an electrical resistance heating element. Because the rigid rod 4 is to be rotated, the electrical contacts that supply the rigid rod 4 in such an embodiment must be formed by suitable slide contacts.

In the shown embodiment the switch button 7 is adapted to turn on and off both the electrical motor 9 and the heating means 10. In alternative embodiments the electrical motor 9 and the heating means 10 are turned on and off by means of separate switches, hence making it possible to use the eye treatment device with only one of these turned on.

In the same way the electrical source for both the electrical motor 9 and the heating means 10 is the batteries in battery section 8.

The switch button 7 is arranged in such a way that the electrical motor 9 may be rotated in both rotational directions.

As shown in figs. 1 to 3 the treatment member 3 consists of a cone of sponge. In the shown embodiment the treatment member 3 of the swab 4 is replaceable as the treatment member 3 quite simple may be twisted on and off the rigid rod 4. Hereby it is easy and also cheap to replace the treatment member 3, when it is worn or too dirty to be used.

The treatment member 3 of the swab 2 is selected to consist of a cone of sponge in order to be able to absorb a treatment liquid that may be applied tp the swab prior to use. In this way, the eye disease can be treated more efficiently and the person with eye disease can be cured faster.

In fig. 4 is shown how the eye treatment device 1 according to the invention is used for treating a lower eyelid 12 of an eye 13 for an eye disease. The user activate the eye treatment device by pressing the button 7, whereby the swab are heated and begins to rotate. When the swab is heated to the predetermined temperature of e.g. between 40 to 43 °C, in a short heating period of e.g. 30 second, the user can easily move or slide the eye treatment device 1 from one side of the eye 13 to the other side in such a way the swab 3 slides over the lower eyelid 12 while simultaneously rotating and heating up and pushing away debris and various crusts from the eyelid 12. In this way the debris are heated to and thus softened, and as the swab simultaneously rotates the debris are removed in the same process step. If electrical motor 9 is rotating at a speed of about 108 rpm, which means that the swab 2 is rotating at the same speed.

The eye 13 also comprises an upper eyelid 14 and a plurality of eyelashes 15, which parts of the eye may be treated in the same way as the lower eyelid 12.

By adding a disinfecting liquid, such as a saline solution, to the treatment member 3, the softening and loosening process of the debris, crusts and/or cysts etc. is facilitated. This is due to the heating of the swab 2, the disinfecting liquid is also heated, which greatly promotes the softening and loosening process of the debris, crusts and/or cysts and thus also the cleaning process of the eye 13.

The eye also comprises an eyeball 16, which should not be touched during the treatment of the eye because it will feel uncomfortable to the person with eye disease. Because the treatment member 3 consists of a cone, it is easy to avoid that the treatment member 3 comes into contact with the eyeball 16. In this way, by using the eye treatment device according to the invention two process steps of softening and scrubbing the debris, crusts and/or cysts may be done in a single process step.

The device according to the invention has a simple and inexpensive design, and can therefore be used equally well for both privately and in medical or hospital facilities where the known eye treatment devices are too troublesome and complicated to use. The device may be used for treating various eye diseases e.g. meibomian gland dysfunction, blepharitis, and dry eye syndrome, however, the device may be equally well used for a simple hygienic treatment of the eye-regions.

## Claims

1. An eye treatment device (1) arranged for removing debris from margin of the eyelid and/or for massaging the meibomian glands, said eye treatment device (1) comprises a swab (3) operatively connected to a housing (5) that accommodates a drive unit (9), said drive unit being arranged for moving the swab (3) relative to said housing (5), and wherein said eye treatment device comprises heating means (10) arranged for heating the swab (3), **characterized in that**
- the moving of the swab (3) relative to said housing (5) is a rotating movement,
- the swab (2) is made of cotton wadding or sponge material,
- said swab is arranged for absorbing a treatment liquid, and
- wherein said swab is arranged for being releasably attached to the housing (5), or to an extension member (4) arranged for coupling the swab (3) to the housing (5) .

2. An eye treatment device (1) according to claim 1, wherein the eye treatment device (1) is arranged for heating the swap (3) to a predefined temperature between 33°C and 45°C, preferably between 40°C - 43°C or about 34 °C.

3. An eye treatment device (1) according to claim 1 or 2, wherein the eye treatment device comprises temperature controlling means (10) arranged to heat the temperature of the swap (3) to the predefined temperature and/or maintain the swap at said temperature.

4. An eye treatment device (1) according to claim 3, wherein the temperature controlling means (10) comprises a temperature adjustment means arranged for allowing adjustment of the temperature of the swap during use.

5. An eye treatment device (1) according to any of the preceding claims, wherein the eye treatment device further comprises an ultrasonic means.

6. An eye treatment device (1) according to any of the preceding claims, wherein the extension member is a substantially rigid rod made of a heat conductive material.

7. An eye treatment device (1) according to any of the preceding claims, that the electrical motor (9) of the drive unit is rotatable in both directions.

8. An eye treatment device (1) according to any of the preceding claims, wherein the eye treatment device (1) is arranged for rotating the swab at a speed between 50 and 150 rpm, preferably between 100 and 110 rpm.

9. An eye treatment device (1) according to any of the preceding claims, wherein the eye treatment device comprises rotation control means arranged for allowing adjustment of the rotational speed during use.

10. An eye treatment device (1) according to any of the preceding claims, wherein the swab (2) has a substantially tubular shape with a rounded end.

11. An eye treatment device (1) according to claim 10, wherein the diameter of the tubular shape is between 0.2 cm and 1.0 cm, and preferably around 0.5 cm.

12. An eye treatment device (1) according to any of the preceding claims, **characterized in that** the heating means (10) comprises electrical heating means and an electrical source (8) for supplying electricity to the electrical heating means.

13. An eye treatment device (1) according to any of the preceding claims, wherein the drive unit (9) for moving the swab (2) relative to the drive unit (9) comprises an electrical motor (9) and an electric source (8) for supplying electricity to the electric motor.

## Patentansprüche

1. Augenbehandlungsvorrichtung (1), die zum Entfernen von Schmutz vom Rand des Augenlids und/oder zum Massieren der Meibomusdrüsen eingerichtet ist, wobei die Augenbehandlungsvorrichtung (1) einen Tupfer (3) umfasst, der mit einem Gehäuse (5) wirkverbunden ist, das eine Antriebseinheit (9) aufnimmt, wobei die Antriebseinheit zum Bewegen des Tupfers (3) relativ zu dem Gehäuse (5) angeordnet ist, und wobei die Augenbehandlungsvorrichtung Heizmittel (10) umfasst, die zum Erwärmen des Tupfers (3) angeordnet sind, **dadurch gekennzeichnet, dass**
- das Bewegen des Tupfers (3) relativ zu dem Gehäuse (5) eine Drehbewegung ist,
- der Tupfer (2) aus Baumwollwatte oder Schwammmaterial besteht,
- der Tupfer zum Absorbieren einer Behandlungsflüssigkeit eingerichtet ist, und
- wobei der Tupfer zum lösbaren Anbringen an das Gehäuse (5) oder an ein Verlängerungselement (4) eingerichtet ist, das zum Koppeln des Tupfers (3) an das Gehäuse (5) eingerichtet ist.

2. Augenbehandlungsvorrichtung (1) nach Anspruch 1, wobei die Augenbehandlungsvorrichtung (1) zum Erwärmen des Tauschers (3) auf eine vordefinierte Temperatur zwischen 33°C und 45°C, vorzugsweise zwischen 40°C - 43°C oder etwa 34°C, eingerichtet ist.

3. Augenbehandlungsvorrichtung (1) nach Anspruch 1 oder 2, wobei die Augenbehandlungsvorrichtung Temperatursteuermittel (10) umfasst, die zum Erwärmen der Temperatur des Tauschers (3) auf die vordefinierte Temperatur und/oder Aufrechterhalten des Tauschers auf der Temperatur eingerichtet sind.

4. Augenbehandlungsvorrichtung (1) nach Anspruch 3, wobei das Temperatursteuermittel (10) ein Temperatureinstellmittel umfasst, das zum Ermöglichen einer Einstellung der Temperatur des Tauschers während des Gebrauchs eingerichtet ist.

5. Augenbehandlungsvorrichtung (1) nach einem der vorhergehenden Patentansprüche, wobei die Augenbehandlungsvorrichtung ferner ein Ultraschallmittel umfasst.

6. Augenbehandlungsvorrichtung (1) nach einem der vorhergehenden Patentansprüche, wobei das Verlängerungselement ein im Wesentlichen starrer Stab ist, der aus einem wärmeleitfähigen Material besteht.

7. Augenbehandlungsvorrichtung (1) nach einem der vorhergehenden Patentansprüche, wobei der elektrische Motor (9) der Antriebseinheit in beide Richtungen drehbar ist.

8. Augenbehandlungsvorrichtung (1) nach einem der vorhergehenden Patentansprüche, wobei die Augenbehandlungsvorrichtung (1) zum Drehen des Tupfers mit einer Geschwindigkeit zwischen 50 und 150 U/min, vorzugsweise zwischen 100 und 110 U/min, eingerichtet ist.

9. Augenbehandlungsvorrichtung (1) nach einem der vorhergehenden Patentansprüche, wobei die Augenbehandlungsvorrichtung Drehsteuermittel umfasst, die zum Ermöglichen einer Einstellung der Drehgeschwindigkeit während des Gebrauchs eingerichtet sind.

10. Augenbehandlungsvorrichtung (1) nach einem der vorhergehenden Patentansprüche, wobei der Tupfer (2) eine im Wesentlichen rohrförmige Form mit einem abgerundeten Ende aufweist.

11. Augenbehandlungsvorrichtung (1) nach Anspruch 10, wobei der Durchmesser der rohrförmigen Form zwischen 0,2 cm und 1,0 cm und vorzugsweise etwa 0,5 cm beträgt.

12. Augenbehandlungsvorrichtung (1) nach einem der vorhergehenden Patentansprüche, **dadurch gekennzeichnet, dass** das Heizmittel (10) elektrische Heizmittel und eine elektrische Quelle (8) zum Zuführen von Elektrizität zu den elektrischen Heizmitteln umfasst.

13. Augenbehandlungsvorrichtung (1) nach einem der vorhergehenden Patentansprüche, wobei die Antriebseinheit (9) zum Bewegen des Tupfers (2) relativ zu der Antriebseinheit (9) einen elektrischen Motor (9) und eine elektrische Quelle (8) zum Zuführen von Elektrizität zu dem elektrischen Motor umfasst.

## Revendications

1. Un dispositif de traitement oculaire (1) qui est conçu pour le retrait de débris à partir du bord de la paupière et/ou pour le massage des glandes de Meibomius ; dans lequel le dispositif de traitement oculaire (1) en question comprend un coton-tige (3) qui est relié d'une manière opérationnelle à un logement (5) dans lequel vient s'insérer une unité d'entraînement (9), l'unité d'entraînement en question étant conçue pour mettre le coton-tige (3) en mouvement par rapport audit logement (5) ; et dans lequel ledit dispositif de traitement oculaire comprend un moyen de chauffage (10) qui est conçue pour chauffer le coton-tige (3), **caractérisé en ce que** :
- la mise en mouvement du coton-tige (3) par rapport au logement (5) en question correspond à un mouvement rotatif ;
- le coton-tige (2) est réalisé à partir d'ouate de coton ou d'une matière spongieuse ;
- ledit coton-tige est conçu pour absorber un liquide de traitement ; et
- dans lequel ledit coton-tige est conçu pour venir se fixer de manière amovible au logement (5) ou à un élément sous la forme d'une extension (4) qui est conçu pour l'accouplement du coton-tige (3) au logement (5).

2. Un dispositif de traitement oculaire (1) conformément à la revendication 1, dans lequel le dispositif de traitement oculaire (1) est conçu pour chauffer le coton-tige (3) jusqu'à une température prédéfinie qui se situe entre 33 °C et 45 °C, de préférence entre 40 °C et 43 °C ou qui s'élève à environ 34 °C.

3. Un dispositif de traitement oculaire (1) conformément à la revendication 1 ou 2, dans lequel le dispositif de traitement oculaire comprend un moyen de réglage de la température (10) qui est conçu pour élever la température du coton-tige (3) jusqu'à la température qui a été prédéfinie et/ou pour maintenir le coton-tige à la température en question.

4. Un Dispositif de traitement oculaire (1) conformément à la revendication 3, dans lequel le moyen de réglage de la température (10) comprend un moyen d'ajustement de la température qui est conçu pour permettre un ajustement de la température du coton-tige au cours de l'utilisation de ce dernier.

5. Un dispositif de traitement oculaire (1) conformément à l'une quelconque des revendications précédentes, dans lequel le dispositif de traitement oculaire comprend en outre un moyen ultrasonore.

6. Un dispositif de traitement oculaire (1) conformément à l'une quelconque des revendications précédentes, dans lequel l'élément sous la forme d'une extension représente une tige essentiellement rigide réalisée à partir d'un matériau thermoconducteur.

7. Un dispositif de traitement oculaire (1) conformément à l'une quelconque des revendications précédentes, dans lequel le moteur électrique (9) de l'unité d'entraînement est à même d'effectuer des rotations dans les deux directions.

8. Un dispositif de traitement oculaire (1) conformément à l'une quelconque des revendications précédentes, dans lequel le dispositif de traitement oculaire (1) est conçu pour la mise en rotation du coton-tige à une vitesse qui se situe entre 50 et 150 tours/minute, de préférence entre 100 et 110 tours/minute.

9. Un dispositif de traitement oculaire (1) conformément à l'une quelconque des revendications précédentes, dans lequel le dispositif de traitement oculaire comprend un moyen de réglage de la rotation qui est conçu pour permettre un ajustement de la vitesse de rotation au cours de son utilisation.

10. Un dispositif de traitement oculaire (1) conformément à l'une quelconque des revendications précédentes, dans lequel le coton-tige (2) possède une configuration essentiellement de forme tubulaire avec une extrémité arrondie.

11. Un dispositif de traitement oculaire (1) conformément à la revendication 10, dans lequel le diamètre de la configuration de forme tubulaire se situe entre 0,2 cm et 1,0 cm, et de préférence s'élève à environ 0,5 cm.

12. Un dispositif de traitement oculaire (1) conformément à l'une quelconque des revendications précédentes, **caractérisé en ce que** le moyen de chauffage (10) comprend un moyen de chauffage électrique et une source d'électricité (8) à des fins d'alimentation de l'électricité au moyen de chauffage électrique.

13. Un dispositif de traitement oculaire (1) conformément à l'une quelconque des revendications précédentes, dans lequel l'unité d'entraînement (9) qui est destinée à la mise en mouvement du coton-tige (2) par rapport à l'unité d'entraînement (9) comprend un moteur électrique (9) et une source électrique (8) destinée à alimenter le moteur électrique en électricité.
